# EUROPÄISCHE PATENTSCHRIFT

(11) **EP 3 909 643 B1**
(45) Veröffentlichungstag und Bekanntmachung des Hinweises auf die Patenterteilung: **25.12.2024**
(21) Anmeldenummer: 20174076.8
(22) Anmeldetag: 12.05.2020
(51) Int. Cl.: A61N 5/10, A61B 6/04

(54) **PATIENTENPOSITIONIERVORRICHTUNG**
PATIENT POSITIONING DEVICE
DISPOSITIF DE POSITIONNEMENT D'UN PATIENT

(43) Veröffentlichungstag der Anmeldung: 17.11.2021
(73) Patentinhaber: BEC GmbH, 72793 Pfullingen (DE)
(72) Erfinder: BUCK, Matthias, 72764 Reutlingen (DE)
(74) Vertreter: Reinhardt, Annette

(56) Entgegenhaltungen:
- DE-B3- 102006 020 868
- US-B2- 10 492 736
- ALLGOWER C E ET AL: "Experiences with an application of industrial robotics for accurate patient positioning in proton radiotherapy", INTERNATIONAL JOURNAL OF MEDICAL ROBOTICS AND COMPUTER ASSISTEDSURGERY, JOHN WILEY, CHICHESTER, GB, vol. 3, 1 March 2007 (2007-03-01), pages 72 - 81, XP002453016, ISSN: 1478-5951, DOI: 10.1002/RCS.128

## Beschreibung

Die Erfindung betrifft eine Patientenpositioniervorrichtung der im Oberbegriff des Anspruchs 1 angegebenen Gattung.

Aus der EP 1 985 237 A1 ist eine Patientenpositioniervorrichtung bekannt, die einen Roboterarm und eine an dem Roboterarm oder an der Patientenliege gehaltene Patientenaufnahme aufweist.

Die US 10,492,736 B2 offenbart eine Abschirmung für einen Bediener einer Röntgeneinrichtung, die beweglich an der Röntgenquelle angeordnet ist.

Die Veröffentlichung Allgöwer et al: "Experiences with an application of industrial robotics for accurate patient positioning in proton radiotherapy", International Journal of Medical Robotics and Computer Assisted Surgery, John Wiley, Chichester, GB; Bd. 3, 1. März 2007, Seiten 72 bis 81 offenbart eine Patientenpositioniervorrichtung mit einem Roboterarm. Die Steuerung des Roboterarms soll im Eingangsbereich des Behandlungsraums angeordnet sein, um sie vor direkter Bestrahlung zu schützen.

Die DE 10 2006 020 868 B3 offenbart eine Patientenpositioniervorrichtung, die einen Roboterarm umfasst.

Bei der Bestrahlung von Tumorgewebe kommt ionisierende Strahlung zum Einsatz. Insbesondere die in der Bor-Neutroneneinfangtherapie (BNCT) zum Einsatz kommende Strahlung ist sehr aggressiv und schädigt insbesondere elektronische Komponenten. Zum Schutz elektronischer Komponenten vor ionisierender Strahlung sind Abschirmeinrichtungen bekannt, die beispielsweise in Form von Platten oder Folie kommerziell erhältlich sind.

Der Erfindung liegt die Aufgabe zugrunde, eine Patientenpositioniervorrichtung der gattungsgemäßen Art zu schaffen, die in einem Umfeld mit ionisierender Strahlung einsetzbar ist und die einfach aufgebaut ist.

Diese Aufgabe wird durch eine Patientenpositioniervorrichtung mit den Merkmalen des Anspruchs 1 gelöst.

Für Patientenpositioniervorrichtungen zum Einsatz kommende Roboterarme sind häufig keine Spezialanfertigungen für die Patientenpositionierung, sondern übliche, in der industriellen Fertigung eingesetzte Roboterarme. Derartige Roboterarme weisen keine Abschirmung gegen ionisierende Strahlung auf. Um dennoch einen solchen Roboterarm für die Patientenpositioniervorrichtung einsetzen zu können, sieht die vorliegende Erfindung eine Gehäuseanordnung für den Roboterarm vor, die mindestens eine Gehäuseeinheit umfasst, die den Roboterarm mindestens teilweise umgibt. Die Gehäuseeinheit ist an dem Roboterarm festgelegt. Die Gehäuseeinheit bewegt sich im Betrieb dadurch mit dem Roboterarm. Die Patientenpositioniervorrichtung weist eine Abschirmung gegen ionisierende Strahlung auf, wobei mindestens ein Teil der Abschirmung an der Gehäuseeinheit angeordnet ist. Erfindungsgemäß ist demnach vorgesehen, die Abschirmung nicht unmittelbar an dem Roboterarm selbst anzuordnen, sondern an einer Gehäuseeinheit, die den Roboterarm zumindest teilweise umgibt.

Dadurch, dass die Abschirmung nicht an dem Roboterarm, sondern an der den Roboterarm umgebenden Gehäuseeinheit angeordnet ist, kann die Abschirmung zumindest teilweise mit Abstand zu dem Roboterarm angeordnet werden. Dies ist insbesondere im Bereich von Antrieben, die sich im Betrieb erwärmen, vorteilhaft. Durch den Abstand wird zum einen eine Beschädigung der Abschirmung durch die Wärme verhindert und zum anderen kann eine ausreichende Luftzirkulation um sich erwärmende Teile des Roboterarms, beispielsweise um die Antriebe, gewährleistet werden.

Dadurch, dass die Abschirmung nicht unmittelbar an dem Roboterarm angeordnet ist, ist eine Gestaltung der Abschirmung mit von dem Roboterarm abweichender Geometrie möglich. Die Geometrie der Gehäuseeinheit kann auf die geometrischen Anforderungen der Abschirmung angepasst werden. Die mindestens eine Gehäuseeinheit kann so gestaltet werden, dass die Abschirmung einfach an der Gehäuseeinheit angebracht werden kann, beispielsweise in Form einer Folie oder einer oder mehrerer Platten. Die Gehäuseeinheit weist hierzu vorteilhaft eine glatte Geometrie mit weichen Übergängen und keinen oder wenigen Kanten auf. Besonders vorteilhaft zur Aufbringung einer Abschirmung ist eine großflächige Geometrie. Vorteilhaft sind beispielsweise Geometrien, die sich an einen Quader mit abgerundeten Ecken, einen Kegelstumpf, einen Pyramidenstumpf mit abgerundeten Ecken, eine Kugel, ein Ellipsoid, eine Halbkugel, einen Zylinder oder andere geometrische Grundformen anlehnen.

Vorteilhaft umfasst die Gehäuseeinheit zumindest einen abnehmbaren Deckel. Vorteilhaft ist mindestens ein Teil der Abschirmung an dem Deckel gehalten und mit dem Deckel abnehmbar. Dadurch kann die Gehäuseeinheit so gestaltet werden, dass zu wartende oder zu reparierende Komponenten des Roboterarms einfach durch Abnahme des Deckels zugänglich sind. Dadurch, dass die Abschirmung an dem Deckel gehalten und mit diesem abnehmbar ist, ist eine Beschädigung der Abschirmung beispielsweise bei Wartungsarbeiten an dem Roboterarm sicher vermieden. Dadurch wird eine lange Lebensdauer der üblicherweise sehr teuren Abschirmung ermöglicht. Dadurch, dass die Abschirmung nicht unmittelbar an dem Roboterarm angeordnet, beispielsweise auf diesen aufgeklebt ist, sondern stattdessen zumindest teilweise mit einem Deckel der Gehäuseeinheit abnehmbar ist, wird vermieden, dass die Abschirmung bei Wartungs- oder Reparaturarbeiten beschädigt werden kann und so teilweise oder vollständig ausgetauscht werden muss.

In vorteilhafter Gestaltung sind alle Teile der Abschirmung für den Roboterarm an der Gehäuseanordnung fixiert. Bevorzugt sind alle Abschnitte der Gehäuseanordnung mit der Abschirmung versehen, insbesondere vollständig mit der Abschirmung ausgekleidet.

In vorteilhafter Gestaltung ist die Abschirmung an der Innenseite der Gehäuseeinheit angeordnet. Dadurch ist die Abschirmung vor äußeren mechanischen Einflüssen und damit einhergehenden möglichen Beschädigungen geschützt.

Vorteilhaft weist der Roboterarm mehrere zueinander bewegliche Abschnitte auf. In vorteilhafter Gestaltung ist für jeden Abschnitt des Roboterarms eine separate Gehäuseeinheit vorgesehen. Dadurch können die Gehäuseeinheiten sich mit den jeweiligen Abschnitten des Roboterarms gemeinsam bewegen, und es wird auf einfache Weise verhindert, dass bei der Bewegung des Roboterarms Bereiche des Roboterarms aus der Gehäuseanordnung heraustreten und dadurch ionisierender Strahlung ausgesetzt werden können.

Ein einfacher Aufbau ergibt sich, wenn mindestens eine Gehäuseeinheit einen an dem Abschnitt des Roboterarms fixierten Grundkörper und einen an dem Grundkörper lösbar gehaltenen Deckel aufweist. Auch mehrere Grundkörper und/oder mehrere Deckel können dabei vorgesehen sein. Es kann auch vorgesehen sein, dass der Grundkörper und/oder der Deckel aus mehreren fest miteinander verbundenen Bauelementen aufgebaut sind. Für die Fixierung des Grundkörpers an dem Roboterarm werden in vorteilhafter Gestaltung ohnehin an dem Roboterarm vorhandene Anschraubpunkte genutzt, so dass eine Fixierung des mindestens einen Grundkörpers an dem Roboterarm auf einfache Weise möglich ist. Der Grundkörper ist vorteilhaft so gestaltet, dass er bei üblichen Wartungs- und Reparaturarbeiten nicht abgenommen werden muss. Daher kommt für die Befestigung des Grundkörpers an dem Roboterarm auch eine aufwendiger zu lösende Befestigung in Betracht. Die Befestigung des Grundkörpers an dem Roboterarm kann vorteilhaft teilweise oder vollständig im Gehäuseinnenraum der Gehäuseeinheit angeordnet sein. Die Befestigung des Grundkörpers an dem Roboterarm kann beispielsweise Bügel oder Klemmprofile umfassen, die in dem Gehäuseinnenraum verlaufen. Der Deckel ist an dem Grundkörper vorteilhaft über einfach von außen zugängliche Befestigungsmittel fixiert. Die Position der Befestigungsmittel kann dabei so gewählt werden, dass eine gute Zugänglichkeit gegeben ist, da die Position der Befestigungsmittel des Deckels an dem Grundkörper unabhängig von der Position eventueller Anschraubpunkte des Roboterarms ist.

Um die Montage des Deckels an dem Grundkörper zu vereinfachen, ist vorteilhaft vorgesehen, dass mindestens eine Gehäuseeinheit Mittel zur Positionierung des Deckels gegenüber dem Grundkörper aufweist. In vorteilhafter Gestaltung sind die Mittel zur Positionierung des Deckels gegenüber dem Grundkörper durch einen umlaufenden Rand gebildet. Auch einzelne Positionierzapfen, Einführschrägen oder dergleichen können als Mittel zur Positionierung des Deckels gegenüber dem Grundkörper vorgesehen sein. Auch eine andere konstruktive Gestaltung der Mittel zur Positionierung des Deckels gegenüber dem Grundkörper kann vorteilhaft sein.

Vorteilhaft ist mindestens eine Gehäuseeinheit mindestens teilweise mit einer Dämpfungsschicht versehen. Eine solche Dämpfungsschicht kann insbesondere dazu vorgesehen sein, Beschädigungen bei einer Kollision des Roboterarms mit anderen Gegenständen zu verhindern. Eine vorteilhafte Gestaltung ergibt sich insbesondere, wenn die Dämpfungsschicht außerhalb der Abschirmung angeordnet ist, die Abschirmung also näher am Gehäuseinnenraum liegt als die Dämpfungsschicht. Dadurch wird die Dämpfungswirkung der Dämpfungsschicht durch die Abschirmung nicht verändert oder beeinträchtigt.

Vorteilhaft ist die Dämpfungsschicht an einer Tragstruktur der Gehäuseeinheit gehalten. Die Tragstruktur ist bevorzugt eine Innenschale der Gehäuseeinheit. In besonders bevorzugter Ausführung ist die Abschirmung an der Innenseite und die Dämpfungsschicht an der Außenseite der Innenschale angeordnet. Auch eine andere Gestaltung der Tragstruktur, beispielsweise in Form von einzelnen Rippen oder in Form einer Gitterstruktur kann vorteilhaft sein. In vorteilhafter Gestaltung ist die Dämpfungsschicht von einer Außenhülle abgedeckt. Dadurch wird ein ansprechendes Äußeres der Patientenpositioniervorrichtung erreicht. Vorteilhaft umfasst die Dämpfungsschicht elastisches Dämpfungsmaterial. Das Dämpfungsmaterial kann beispielsweise Schaumstoff oder dergleichen sein. Die Dämpfungsschicht kann alternativ oder zusätzlich ein oder mehrere gasgefüllte Kissen umfassen. Die Dämpfungswirkung des mindestens einen gasgefüllten Kissens wird durch das eingeschlossene Gas erzielt. Das eingeschlossene Gas ist insbesondere Luft. Das eingeschlossene Gas kann gleichzeitig als Kontaktsensor genutzt werden, beispielsweise, indem Druckschwankungen des Gases gemessen werden. Die Außenhülle kann einen Teil des das Gas umgebenden Kissens bilden. Es kann jedoch auch vorgesehen sein, dass das Gas in einem Kissen angeordnet ist, das seinerseits von einer Außenhülle abgedeckt ist.

Besonders bevorzugt ist in der Dämpfungsschicht mindestens ein Kontaktsensor angeordnet. Der Kontaktsensor erzeugt ein entsprechendes Signal, wenn die Dämpfungsschicht zusammengedrückt wird, beispielsweise aufgrund eines unerwünschten Kontakts mit einem Gegenstand, und kann den Roboterarm entsprechend ansteuern, beispielsweise abschalten. Besonders bevorzugt ist eine Vielzahl von Kontaktsensoren in der Dämpfungsschicht angeordnet. Die Kontaktsensoren weisen zueinander vorteilhaft an jeder Stelle der Dämpfungsschicht einen Abstand von weniger als 10 cm auf. Dadurch kann ein eventueller Kontakt in der Fläche auf einfache Weise ermittelt werden.

Der Roboterarm ist bevorzugt ein Roboterarm mit mindestens fünf, insbesondere mit mindestens sechs Rotationsbewegungsachsen. Der Roboterarm weist vorteilhaft ein Grundgestell, ein an dem Grundgestell um eine erste Rotationsbewegungsachse drehbar gelagertes Karussell, eine an dem Karussell um eine zweite Rotationsbewegungsachse drehbar gelagerte Schwinge, einen an der Schwinge drehbar gelagerten Arm und eine an dem Arm gelagerte Hand auf. Die Hand weist bevorzugt drei Rotationsbewegungsachsen auf. Auch eine andere Gestaltung des Roboterarms kann jedoch vorteilhaft sein.

Ein Ausführungsbeispiel der Erfindung wird im Folgenden anhand der Zeichnung erläutert. Es zeigen:
- Fig. 1: eine Seitenansicht einer Patientenpositioniervorrichtung,
- Fig. 2: eine Seitenansicht der Patientenpositioniervorrichtung aus Fig. 1 in Richtung des Pfeils II in Fig. 1,
- Fig. 3 bis Fig. 6: teilgeschnittene Ansichten der Patientenpositioniervorrichtung aus Fig. 1 und 2, wobei die Gehäuseanordnung teilweise geschnitten dargestellt und dadurch der in der Gehäuseanordnung angeordnete Roboterarm sichtbar ist und wobei die Patientenaufnahme nur in Fig. 6 dargestellt ist,
- Fig. 7 und Fig. 8: schematische Darstellungen der Patientenpositioniervorrichtung entsprechend den Ansichten aus Fig. 1 und Fig. 2, wobei die Gehäuseanordnung mit gestrichelter Linie dargestellt ist und der in der Gehäuseanordnung angeordnete Roboterarm mit durchgezogener Linie und wobei die Patientenaufnahme und die Gehäuseeinheit am Befestigungsflansch nicht dargestellt sind,
- Fig. 9: eine Seitenansicht der Patientenpositioniervorrichtung entsprechend der Ansicht aus Fig. 1, wobei die Patientenaufnahme, die Gehäuseeinheit des Gestells und die Gehäuseeinheit des Befestigungsflanschs nicht dargestellt sind,
- Fig. 10: eine Seitenansicht in Richtung des Pfeils X in Fig. 9,
- Fig. 11: eine Schnittdarstellung entlang der Linie XI-XI in Fig. 10,
- Fig. 12: eine schematische Darstellung des Aufbaus der Patientenpositioniervorrichtung in der Schnittebene aus Fig. 11,
- Fig. 13: die Einzelheit XIII aus Fig. 12 in vergrößerter Darstellung,
- Fig. 14 bis Fig. 16: schematische Darstellungen von unterschiedlichen Befestigungen einer Gehäuseeinheit an einem Roboterarm.

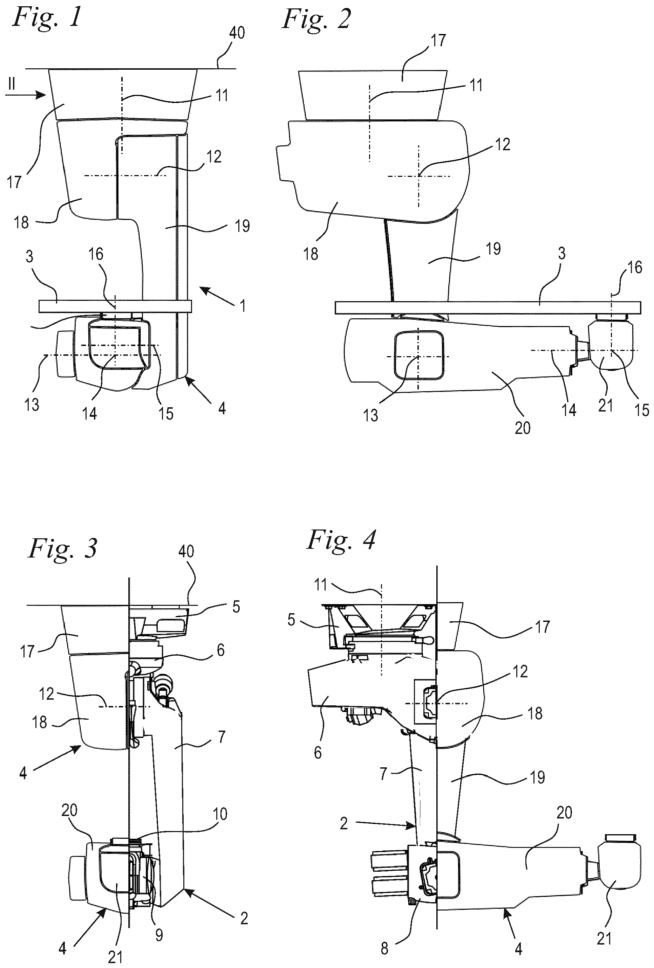

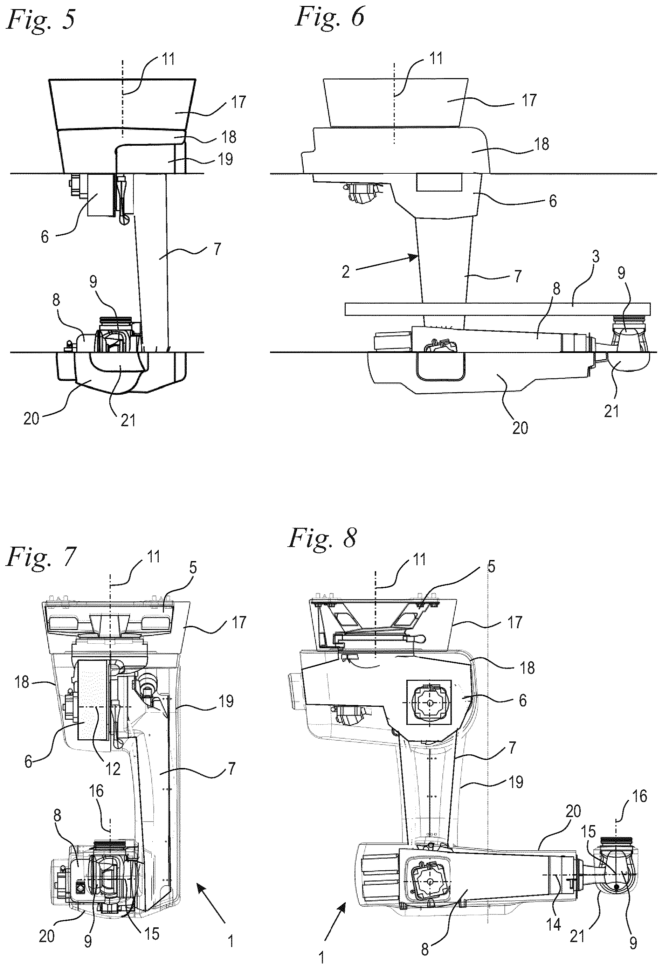

Die Fig. 1 und 2 zeigen eine Patientenpositioniervorrichtung 1. Derartige Patientenpositioniervorrichtungen 1 werden eingesetzt, um Patienten gegenüber einer Bestrahlungseinrichtung zu positionieren. Der von der Bestrahlungseinrichtung emittierte Strahl ist insbesondere ein Strahl aus ionisierender Strahlung. Derartige Bestrahlungseinrichtungen werden insbesondere bei der Tumortherapie eingesetzt. Besonders bevorzugt ist die Bestrahlungseinrichtung eine Bestrahlungseinrichtung für die Bor-Neutroneneinfangtherapie (BNCT - Boron Neutron Capture Therapy). Die Patientenpositioniervorrichtung 1 umfasst einen Roboterarm 2 (Fig. 6), der eine Patientenaufnahme 3 trägt. Im Ausführungsbeispiel ist die Patientenaufnahme 3 eine Patientenliege. Die Patientenaufnahme 3 kann auch in anderer Form ausgebildet sein, beispielsweise als Sitz. Im Ausführungsbeispiel ist die Patientenpositioniervorrichtung 1 an einer Raumdecke 40 fixiert. Auch eine Fixierung auf dem Boden kann vorteilhaft sein.

Wie die Fig. 3 und 4 zeigen, ist der Roboterarm 2 von einer Gehäuseanordnung 4 umgeben. In den Fig. 1 und 2 ist die Gehäuseanordnung 4 vollständig dargestellt und in den Fig. 3 bis 6 teilgeschnitten, so dass der in der Gehäuseanordnung 4 angeordnete Roboterarm 2 sichtbar ist. Der Roboterarm 2 ist, wie Fig. 3 zeigt, unmittelbar an der Raumdecke 40 gehalten. In alternativer Ausführung kann auch vorgesehen sein, dass der Roboterarm 2 an einer Linearachse fixiert ist, die ihrerseits an der Raumdecke 40 gehalten ist. Die Linearachse bildet eine Linearbewegungseinrichtung, die den gesamten Roboterarm 2 entlang einer Raumachse, bevorzugt einer horizontalen Raumachse, bewegt. Dadurch wird der Bewegungsbereich der Patientenpositioniervorrichtung 1 vergrößert. Auch eine am Boden angeordnete Linearachse kann vorteilhaft sein. Auch eine andere Linearbewegungseinrichtung kann vorgesehen sein. Ist der Roboterarm 2 an einer Linearbewegungseinrichtung gehalten, so kann der Roboterarm 2 bei einer Strahlenbehandlung so positioniert werden, dass möglichst wenig ionisierende Strahlung auf den Roboterarm 2 trifft.

Der Roboterarm 2 weist, wie die Fig. 3 und 4 zeigen, ein Grundgestell 5 auf. Der Roboterarm 2 weist außerdem ein Karussell 6, eine Schwinge 7, einen Arm 8 und eine Hand 9 auf. Die Hand 9 weist einen Befestigungsflansch 10 auf, an dem die Patientenaufnahme 3 fixiert ist.

Das Grundgestell 5 kann an der Raumdecke 40 oder alternativ an einer Linearachse oder dem Boden eines Raums fixiert sein. An dem Grundgestell 5 ist das Karussell 6 um eine erste Rotationsbewegungsachse 11 drehbar gelagert. Die erste Rotationsbewegungsachse 11 ist im Ausführungsbeispiel vertikal ausgerichtet. Am Karussell 6 ist die Schwinge 7 um eine zweite Rotationsbewegungsachse 12 drehbar gelagert. Die zweite Rotationsbewegungsachse 12 ist, wie die Fig. 1 bis 3 zeigen, im Ausführungsbeispiel horizontal ausgerichtet. Die Schwinge 7 ist länglich ausgebildet. An einem Ende der Schwinge 7 ist die zweite Rotationsbewegungsachse 12 angeordnet und an einem zweiten Ende der Schwinge 7 eine dritte Rotationsbewegungsachse 13. Im Ausführungsbeispiel verläuft die dritte Rotationsbewegungsachse 13 parallel zur zweiten Rotationsbewegungsachse 12. An der dritten Rotationsbewegungsachse 13, die in den Fig. 1 und 2 dargestellt ist, ist der Arm 8 (Fig. 4 und 6) des Roboterarms 2 schwenkbar gelagert. Der Arm 8 ist länglich ausgebildet und im Bereich eines ersten Endes um die dritte Rotationsbewegungsachse 13 an der Schwinge 7 drehbar gelagert. Der Arm 8 trägt im Bereich seines anderen Endes die Hand 9. Die Hand 9 ist, wie die Fig. 1, 2 und 8 zeigen, am Arm 8 um eine vierte Rotationsbewegungsachse 14 drehbar gelagert. Im Ausführungsbeispiel ist die vierte Rotationsbewegungsachse 14 in Längsrichtung des Arms 8 ausgerichtet. Die Hand 9 weist eine fünfte Rotationsbewegungsachse 15 auf, die senkrecht zur vierten Rotationsbewegungsachse 14 ausgerichtet ist, sowie eine sechste Rotationsbewegungsachse 16, die senkrecht zur fünften Rotationsbewegungsachse 15 verläuft. Die Hand 9 weist den Befestigungsflansch 10 auf, der in den Fig. 5 und 6 sichtbar ist und an dem die Patientenaufnahme 3 fixiert ist, wie Fig. 6 zeigt.

Das Grundgestell 5, das Karussell 6, die Schwinge 7, der Arm 8 sowie die Hand 9 bilden zueinander bewegliche Abschnitte des Roboterarms 2. Die Gehäuseanordnung 4 umfasst mehrere Gehäuseeinheiten 17 bis 22, die unterschiedliche Abschnitte des Roboterarms 2 umgeben. Wie die Fig. 1 bis 6 zeigen, ist eine erste Gehäuseeinheit 17 vorgesehen, die das Grundgestell 5 umgibt. Eine zweite Gehäuseeinheit 18 umgibt das Karussell 6. Eine dritte Gehäuseeinheit 19 umgibt die Schwinge 7. Eine vierte Gehäuseeinheit 20 umgibt den Arm 8. Eine fünfte Gehäuseeinheit 21 umgibt die Hand 9. Eine sechste Gehäuseeinheit 22 umgibt den Befestigungsflansch 10. Für die zueinander beweglichen Abschnitte des Roboterarms 2 sind demnach unterschiedliche Gehäuseeinheiten 17 bis 22 der Gehäuseanordnung 4 vorgesehen. Die Gehäuseeinheiten 17 bis 22 sind so ausgebildet, dass die Bewegung des Roboterarms 2 durch die Gehäuseeinheiten 17 bis 22 nicht behindert wird. Die Gehäuseeinheiten 17 bis 22 sind gleichzeitig so ausgebildet, dass in jeder Stellung des Roboterarms 2 der Roboterarm 2 weitgehend, insbesondere vollständig von der Gehäuseanordnung 4 umgeben ist. Die ionisierende Strahlung kann vorteilhaft nur durch die Gehäuseanordnung 4 zum Roboterarm 2 gelangen. Die Gehäuseeinheiten 17 bis 22 umgeben die Abschnitte des Roboterarms 2 jeweils nicht vollständig. Die Gehäuseeinheiten 17 bis 22 weisen Öffnungen auf, durch die die Abschnitte des Roboterarms 2 miteinander verbunden sind.

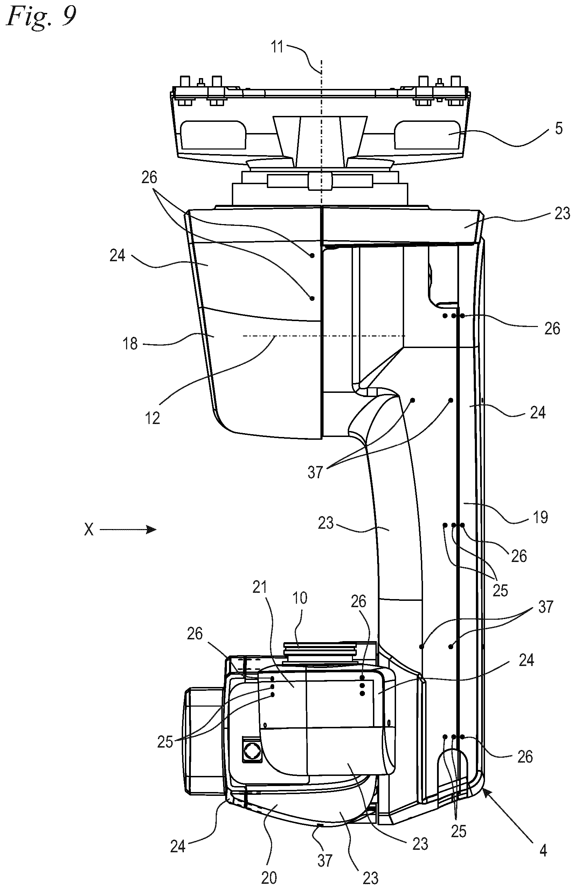

Fig. 9 zeigt die Gehäuseanordnung 4 im Einzelnen. Dabei sind die Gehäuseeinheiten 17 und 22 nicht dargestellt.

Wie Fig. 9 zeigt, weist die Gehäuseeinheit 18 einen Grundkörper 23 sowie einen Deckel 24 auf. Der Deckel 24 ist vorteilhaft mit Befestigungsschrauben 26 am Grundkörper 23 fixiert. Dadurch kann der Deckel 24 abgenommen werden. Der Grundkörper 23 ist vorteilhaft am Roboterarm 2 selbst fixiert. Auch die Gehäuseeinheit 19, die die Schwinge 7 umgibt, weist einen Grundkörper 23 sowie einen Deckel 24 auf. Der Grundkörper 23 ist beispielsweise mit Befestigungsschrauben 37 am Roboterarm 2 fixiert. Die Befestigungsschrauben 37 sind vorzugsweise in ohnehin vorhandene Einschraubpunkte am Roboterarm 2 eingeschraubt. Die Befestigungsschrauben 26 sind jeweils benachbart zu zwei Befestigungsschrauben 25 am Grundkörper 23 angeordnet.

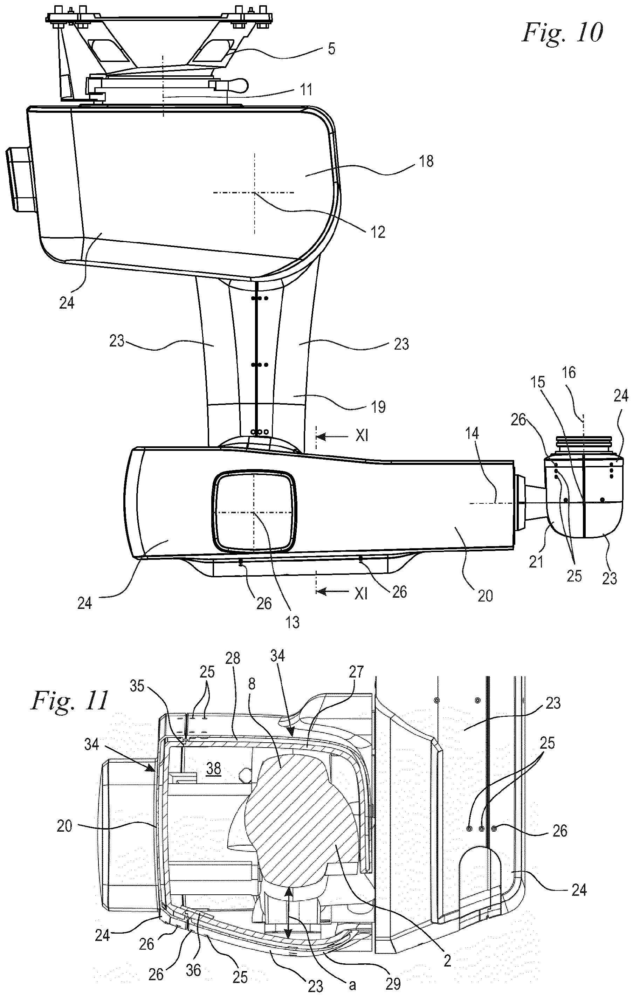

Innerhalb der Gehäuseeinheit 19 verläuft im Ausführungsbeispiel ein in Fig. 11 sichtbarer Befestigungssteg 36, in den die Befestigungsschrauben 25 und 26 eingeschraubt sind. Dadurch ist der Deckel 24 am Grundkörper 23 fixiert. Auch eine andere Fixierung des Deckels 24 am Grundkörper 23, beispielsweise durch in den Grundkörper 23 eingeschraubte Befestigungsschrauben oder über andere Befestigungsmittel, kann vorteilhaft sein.

In entsprechender Weise sind die Gehäuseeinheiten 20 und 21 jeweils aus einem Grundkörper 23 und einem Deckel 24 aufgebaut. Die Deckel 24 sind jeweils so angeordnet, dass zu wartende Komponenten des Roboterarms 2 wie beispielsweise elektrische Antriebe, Kabelverbindungen oder dergleichen nach Abnahme des Deckels 24 gut zugänglich sind.

Wie Fig. 10 zeigt, ist die Gehäuseeinheit 19, die die Schwinge 7 umgibt, im Ausführungsbeispiel zweischalig ausgebildet. Die beiden Schalen des Grundkörpers 23 umschließen die Lagerstellen, an denen die Schwinge 7 am Karussell 6 bzw. am Arm 8 schwenkbar gelagert ist. Der Deckel 24 der dritten Gehäuseeinheit 19 und der Deckel 24 der vierten Gehäuseeinheit 20, die den Arm 8 umgibt, sind von gegenüberliegenden Seiten auf die zugehörigen Grundkörper 23 aufgesetzt. Die sechste Gehäuseeinheit 22 (Fig. 1), die den Befestigungsflansch 10 umgibt, kann am Deckel 24 der fünften Gehäuseeinheit 21 angeformt sein oder separat ausgebildet sein. Die sechste Gehäuseeinheit ist vorteilhaft nicht aus Grundkörper und Deckel aufgebaut, sondern mit dem Deckel 24 der fünften Gehäuseeinheit 21 vollständig abnehmbar. Vorteilhaft sind zumindest die Gehäuseeinheiten 18, 19 und 20, die das Karussell 6, die Schwinge 7 und den Arm 8 umgeben, aus Grundkörper 23 und Deckel 24 aufgebaut.

Wie die Schnittdarstellung in Fig. 11 zeigt, weist der Roboterarm 2 in der dargestellten Schnittdarstellung durch den Arm 8 des Roboterarms 2 über seinen gesamten Umfang einen Abstand a zur Gehäuseanordnung 4 auf. Der Roboterarm 2 besitzt eine vergleichsweise unregelmäßige Außenkontur, die auch scharfe Kanten aufweist. Die Gehäuseanordnung 4 ist dagegen mit gerundeten Kanten und geraden oder gebogenen Konturen ausgebildet. Die Gehäuseeinheit 20 weist eine Gehäusewand 34 auf, die einen Gehäuseinnenraum 38 umgibt. In dem Gehäuseinnenraum 38 ist der Arm 8 des Roboterarms 2 angeordnet. Wie Fig. 11 auch zeigt, ist die Gehäusewand 34 der Gehäuseeinheit 20 mehrschalig ausgebildet. Die weiteren Gehäuseeinheiten 17 bis 19, 21 und 22 der Gehäuseanordnung 4 sind entsprechend aufgebaut. Die Gehäuseeinheiten 17 bis 22 weisen jeweils eine Innenschale 27 auf. Die Innenschale 27 bildet im Ausführungsbeispiel die Tragstruktur der Gehäuseeinheiten 17 bis 22. Im Ausführungsbeispiel sind die Innenschalen 27 mit Ausnahme von Öffnungen in der Gehäusewand 34, die als Durchtrittsöffnung zur Verbindung der Abschnitte des Roboterarms 2 miteinander dienen, geschlossen ausgebildet. Auch eine rippenförmige Gestaltung der Tragstruktur kann jedoch vorgesehen sein. Die Gehäusewand 34 der Gehäuseanordnung 40 weist eine Außenhülle 29 auf. Die Außenhülle 29 ist im Ausführungsbeispiel sehr dünn ausgebildet und vorteilhaft elastisch. Zwischen der Innenschale 27 und der Außenhülle 29 ist eine Dämpfungsschicht 28 angeordnet, deren Komponenten in Fig. 11 nicht dargestellt sind.

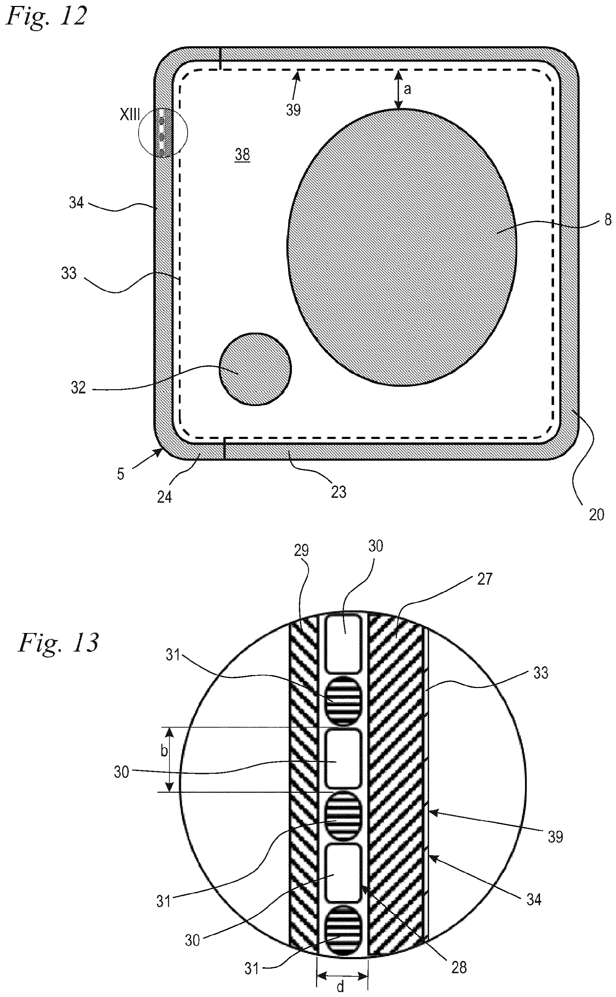

Fig. 12 zeigt schematisch die Anordnung des Roboterarms 2, in der Figur des Arms 8, in der Gehäuseanordnung 4. Der Roboterarm 2 weist allseitig einen Abstand a zur Gehäusewand 34 auf. Die Gehäusewand 34 umschließt einen Gehäuseinnenraum 38. Im Gehäuseinnenraum 38 verläuft im Ausführungsbeispiel neben dem Arm 8 ein Kabelstrang 32. Der Kabelstrang 32 kann beispielsweise Kabel zur Stromversorgung, beispielsweise zur Versorgung der elektrischen Antriebe und Sensoren, oder Datenleitungen zur Übermittlung von Sensorsignalen umfassen. Wie Fig. 12 schematisch zeigt, ist an der den Gehäuseinnenraum 38 begrenzenden Innenseite 39 der Gehäuseeinheit 20 eine Abschirmung 33 angeordnet. Die Abschirmung 33 dient zur Abschirmung gegenüber ionisierender Strahlung. Eine derartige Abschirmung kann beispielsweise als Folie ausgebildet sein. Auch eine Abschirmung in Form von Platten oder dergleichen kann vorgesehen sein. Die Abschirmung 33 ist fest mit der Gehäuseanordnung 4 verbunden. Ein Abschnitt der Abschirmung 33 ist dabei am Grundkörper 23 fixiert und ein anderer Abschnitt der Abschirmung 33 am Deckel 24. In besonders vorteilhafter Gestaltung ist die Abschirmung 33 flächig im Grundkörper 23 und im Deckel 24 eingeklebt.

Fig. 13 zeigt den Aufbau der Gehäusewand 34 im Einzelnen. Von innen nach außen ist die Gehäusewand 34 aus Abschirmung 33, Innenschale 27, Dämpfungsschicht 28 und Außenhülle 29 aufgebaut. In der Dämpfungsschicht 28 ist Dämpfungsmaterial 30 angeordnet. Das Dämpfungsmaterial 30 kann beispielsweise durch Schaumstoffelemente gebildet sein. Zwischen benachbarten Abschnitten von Dämpfungsmaterial 30 erstrecken sich vorteilhaft Kontaktsensoren 31. Die Kontaktsensoren 31 können beispielsweise als Schaltleisten ausgeführt sein. Die Abschirmung 33 ist an einer Innenseite 39 der Gehäusewand 34 angeordnet. Die Dicke d der Dämpfungsschicht 28 ist vorteilhaft vergleichsweise groß. Die Dicke d der Dämpfungsschicht 28 kann beispielsweise 0,5 cm bis 15 cm betragen. Die Dicke d der Dämpfungsschicht 28 beträgt insbesondere von 0,5 cm bis 3 cm. Die Dicke d der Dämpfungsschicht 28 ist vorteilhaft auf die Verfahrgeschwindigkeiten des Roboterarms 2 abgestimmt, wobei für große Verfahrgeschwindigkeiten große Dicken d vorteilhaft sind. Die Außenhülle 29 ist vorteilhaft elastisch, so dass die Dämpfungswirkung der Dämpfungsschicht 28 durch die Außenhülle 29 nur geringfügig beeinträchtigt wird. Bei einem Kontakt von Gegenständen mit der Außenhülle 29 wird die Dämpfungsschicht 28 zusammengedrückt. Dies dämpft die auf den Gegenstand ausgeübten Kräfte. Bei einem Kontakt mit einem Gegenstand schaltet der mindestens eine Kontaktsensor 31. Dadurch wird der Kontakt von der Patientenpositioniervorrichtung 1 erkannt und der Roboterarm 2 kann entsprechend angesteuert, beispielsweise gestoppt werden. Dadurch, dass die Abschirmung 33 an der Innenseite 39 der Gehäusewand 34 angeordnet ist, verändert die Abschirmung 33 die Dämpfungscharakteristik der Dämpfungsschicht 28 nicht. Die Abschirmung 33 ist gleichzeitig vor mechanischen Einflüssen geschützt, so dass eine Beschädigung der Abschirmung 33 vermieden wird.

In alternativer Ausführung ist vorgesehen, dass die Dämpfungsschicht 28 ein oder mehrere gasgefüllte Kissen, insbesondere Luftkissen umfasst, die die Dämpfungswirkung erzeugen. Die Kissen können von einer Außenhülle 29 abgedeckt sein. Alternativ kann der nach außen weisende Teil der Kissen die Außenhülle 29 bilden. In bevorzugter Gestaltung wird der Druck innerhalb der Kissen überwacht und ausgewertet, um einen Kontakt eines Kissens mit einem Gegenstand zu detektieren. Das mindestens eine Kissen bildet damit einen Kontaktsensor. Auch eine Kombination aus gasgefüllten Kissen und Dämpfungsmaterial kann vorteilhaft sein.

Um sicherzustellen, dass jeder Kontakt von der Gehäuseanordnung 4 erkannt wird, beträgt der Abstand b zwischen benachbarten Kontaktsensoren 31 vorteilhaft weniger als 10 cm, insbesondere weniger als 5 cm.

Wie Fig. 11 zeigt, weist der Grundkörper 23 einen umlaufenden Rand 35 auf, der in eine entsprechend geformte Vertiefung des Deckels 24 ragt. Dadurch kann der Deckel 24 gut am Grundkörper 23 positioniert und vorfixiert werden. Dadurch wird die Montage vereinfacht.

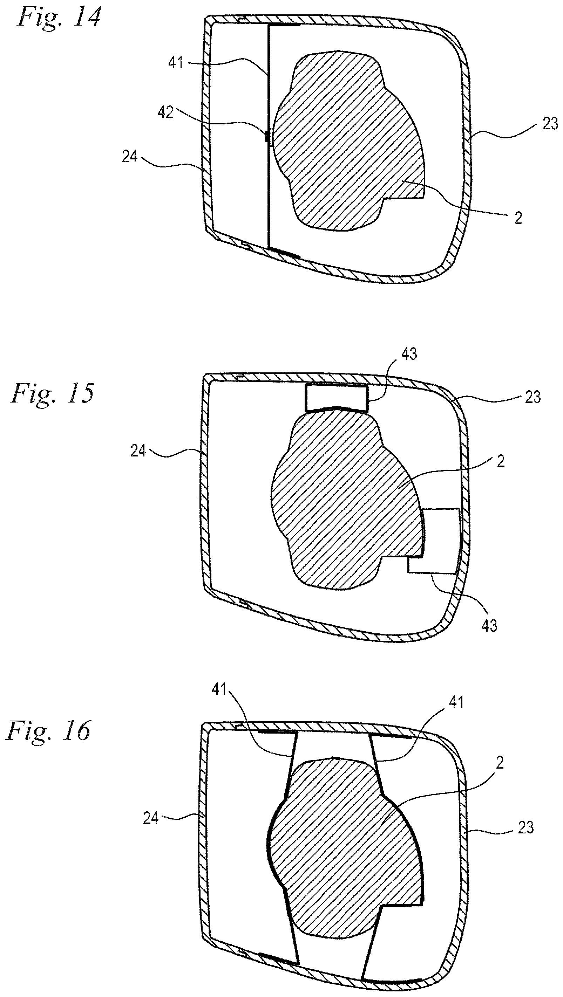

Die Fig. 14 bis 16 zeigen unterschiedliche Varianten der Fixierung des Grundkörpers 23 am Roboterarm 2. Im Ausführungsbeispiel nach Fig. 14 ist ein Bügel 41 vorgesehen, der mit seinen Enden am Grundkörper 23 festgelegt ist. Der Bügel 41 kann beispielsweise am Grundkörper 23 festgeschraubt oder mit diesem verklebt sein. Der Bügel 41 ist über eine oder mehrere Befestigungsschrauben 42 am Roboterarm 2 festgeschraubt. Dadurch fixiert der Bügel 41 den Grundkörper 23 am Roboterarm 2.

Beim Ausführungsbeispiel nach Fig. 15 sind Klemmprofile 43 am Grundkörper 23 fixiert, beispielsweise durch Kleben oder Schrauben. Die Klemmprofile 43 umgreifen den Roboterarm 2 zumindest teilweise an gegenüberliegenden Seiten. Dadurch ist der Roboterarm 2 an den Klemmprofilen 43 formschlüssig fixiert. Zusätzlich können die Klemmprofile 43 am Roboterarm 2 verschraubt sein, insbesondere zur Sicherung der klemmenden Fixierung.

Das Ausführungsbeispiel nach Fig. 16 zeigt eine Gestaltung, bei der am Grundkörper 23 zwei Bügel 41 an gegenüberliegenden Seiten des Roboterarms 2 angeordnet sind. Der Roboterarm 2 ist zwischen den beiden Bügeln 41 geklemmt gehalten. Auch eine zusätzliche Fixierung über eine oder mehrere Befestigungsschrauben kann vorteilhaft sein. Die Bügel 41 können am Grundkörper 23 durch Kleben oder mittels Schrauben fixiert sein. Es kann auch vorgesehen sein, dass die Bügel 41 am Grundkörper 23 aufgrund ihrer Eigenspannung elastisch gehalten sind. Auch andere Befestigungsarten zur Fixierung des Grundkörpers 23 am Roboterarm 2 können vorteilhaft sein.

Im Ausführungsbeispiel ist die Gestaltung der Gehäuseeinheit 20 im Einzelnen dargestellt. Die weiteren Gehäuseeinheiten 17, 18, 19 und 21 sind vorteilhaft entsprechend ausgebildet. Auch die sechste Gehäuseeinheit 22 kann entsprechend ausgebildet sein. Der Aufbau der Gehäusewand 34 ist bevorzugt für alle Gehäuseeinheiten der Gehäuseanordnung 4 gleich. Auch für die Fixierung der Grundkörper 23 am Roboterarm 2 kommen für alle Gehäuseeinheiten 17 bis 22 alle beschriebenen Fixierungsvarianten in Betracht. Auch andere Arten der Fixierung können vorteilhaft sein.

## Patentansprüche

1. Patientenpositioniervorrichtung, insbesondere für die Bor-Neutroneneinfangtherapie (BNCT), umfassend einen Roboterarm (2) und eine an dem Roboterarm (2) gehaltene Patientenaufnahme (3),
**dadurch gekennzeichnet, dass** die Patientenpositioniervorrichtung (1) eine Gehäuseanordnung (4) für den Roboterarm (2) umfasst, die mindestens eine Gehäuseeinheit (17, 18, 19, 20, 21) umfasst, die den Roboterarm (2) mindestens teilweise umgibt, wobei die Gehäuseeinheit (17, 18, 19, 20, 21) an dem Roboterarm (2) festgelegt ist und dass die Patientenpositioniervorrichtung (1) eine Abschirmung (33) gegen ionisierende Strahlung aufweist, wobei mindestens ein Teil der Abschirmung (33) an der Gehäuseeinheit (17, 18, 19, 20, 21) angeordnet ist.

2. Patientenpositioniervorrichtung nach Anspruch 1,
**dadurch gekennzeichnet, dass** die Gehäuseeinheit (17, 18, 19, 20, 21) zumindest einen abnehmbaren Deckel (24) umfasst und dass mindestens ein Teil der Abschirmung (33) an dem Deckel (24) gehalten und mit dem Deckel (24) abnehmbar ist.

3. Patientenpositioniervorrichtung nach Anspruch 1 oder 2,
**dadurch gekennzeichnet, dass** alle Teile der Abschirmung (33) für den Roboterarm (2) an der Gehäuseanordnung (4) fixiert sind.

4. Patientenpositioniervorrichtung nach einem der Ansprüche 1 bis 3,
**dadurch gekennzeichnet, dass** die Abschirmung (33) an der Innenseite (39) der Gehäuseeinheit (17, 18, 19, 20, 21) angeordnet ist.

5. Patientenpositioniervorrichtung nach einem der Ansprüche 1 bis 4,
**dadurch gekennzeichnet, dass** der Roboterarm (2) mehrere zueinander bewegliche Abschnitte aufweist und für jeden Abschnitt eine separate Gehäuseeinheit (17, 18, 19, 20, 21) vorgesehen ist.

6. Patientenpositioniervorrichtung nach Anspruch 5,
**dadurch gekennzeichnet, dass** mindestens eine Gehäuseeinheit (17, 18, 19, 20, 21) einen an dem Abschnitt fixierten Grundkörper (23) und einen an dem Grundkörper (23) lösbar gehaltenen Deckel (24) aufweist.

7. Patientenpositioniervorrichtung nach Anspruch 6,
**dadurch gekennzeichnet, dass** mindestens eine Gehäuseeinheit (17, 18, 19, 20, 21) Mittel zur Positionierung des Deckels (24) gegenüber dem Grundkörper (23) aufweist, insbesondere einen umlaufenden Rand (35).

8. Patientenpositioniervorrichtung nach einem der Ansprüche 1 bis 7,
**dadurch gekennzeichnet, dass** mindestens eine Gehäuseeinheit (17, 18, 19, 20, 21) zumindest teilweise mit einer Dämpfungsschicht (28) versehen ist.

9. Patientenpositioniervorrichtung nach Anspruch 8,
**dadurch gekennzeichnet, dass** die Dämpfungsschicht (28) an einer Tragstruktur der Gehäuseeinheit (17, 18, 19, 20, 21), insbesondere an einer Innenschale (27) der Gehäuseeinheit (17, 18, 19, 20, 21) gehalten ist.

10. Patientenpositioniervorrichtung nach Anspruch 8 oder 9,
**dadurch gekennzeichnet, dass** die Dämpfungsschicht (28) von einer Außenhülle (29) abgedeckt ist.

11. Patientenpositioniervorrichtung nach einem der Ansprüche 8 bis 10,
**dadurch gekennzeichnet, dass** die Dämpfungsschicht (28) elastisches Dämpfungsmaterial umfasst.

12. Patientenpositioniervorrichtung nach einem der Ansprüche 8 bis 11,
**dadurch gekennzeichnet, dass** in der Dämpfungsschicht (28) mindestens ein Kontaktsensor (31), insbesondere eine Vielzahl von Kontaktsensoren (31) angeordnet sind.

13. Patientenpositioniervorrichtung nach einem der Ansprüche 1 bis 12,
**dadurch gekennzeichnet, dass** der Roboterarm (2) mindestens fünf, insbesondere mindestens sechs Rotationsbewegungsachsen (11, 12, 13, 14, 15, 16) aufweist.

14. Patientenpositioniervorrichtung nach Anspruch 13,
dadurch gekenntzeichnet, dass der Roboterarm (2) ein Grundgestell (5), ein an dem Grundgestell (5) um eine erste Rotationsbewegungsachse (11) drehbar gelagertes Karussell (6), eine an dem Karussell (6) um eine zweite Rotationsbewegungsachse (12) drehbar gelagerte Schwinge (7), einen an der Schwinge (7) drehbar gelagerten Arm (8) und eine an dem Arm (8) gehaltene Hand (9) aufweist, wobei die Hand (9) drei Rotationsbewegungsachsen (14, 15, 16) aufweist.

## Claims

1. Patient positioning device, in particular for boron neutron capture therapy (BNCT), comprising a robotic arm (2) and a patient receptacle (3) which is held on the robotic arm (2),
**characterized in that** the patient positioning device (1) comprises a housing assembly (4) for the robotic arm (2), which comprises at least one housing unit (17, 18, 19, 20, 21) which at least partially surrounds the robotic arm (2), wherein the housing unit (17, 18, 19, 20, 21) is fixedly established on the robotic arm (2), and **in that** the patient positioning device (1) has a shield (33) in relation to ionized radiation, wherein at least one part of the shield (33) is disposed on the housing unit (17, 18, 19, 20, 21).

2. Patient positioning device according to Claim 1,
**characterized in that** the housing unit (17, 18, 19, 20, 21) comprises at least one removable cover (24), and **in that** at least one part of the shield (33) is held on the cover (24) and is removable conjointly with the cover (24).

3. Patient positioning device according to Claim 1 or 2,
**characterized in that** all parts of the shield (33) for the robotic arm (2) are fixed to the housing assembly (4).

4. Patient positioning device according to one of Claims 1 to 3,
**characterized in that** the shield (33) is disposed on the inside (39) of the housing unit (17, 18, 19, 20, 21).

5. Patient positioning device according to one of Claims 1 to 4,
**characterized in that** the robotic arm (2) has a plurality of portions which are movable relative to one another, and one separate housing unit (17, 18, 19, 20, 21) is provided for each portion.

6. Patient positioning device according to Claim 5,
**characterized in that** at least one housing unit (17, 18, 19, 20, 21) has a main body (23) which is fixed to the portion, and one cover (24) which is releasably held on the main body (23).

7. Patient positioning device according to Claim 6,
**characterized in that** at least one housing unit (17, 18, 19, 20, 21) has means, in particular an encircling periphery (35), for positioning the cover (24) relative to the main body (23).

8. Patient positioning device according to one of Claims 1 to 7,
**characterized in that** at least one housing unit (17, 18, 19, 20, 21) is at least partially provided with a damping layer (28).

9. Patient positioning device according to Claim 8,
**characterized in that** the damping layer (28) is held on a support structure of the housing unit (17, 18, 19, 20, 21), in particular on an inner shell (27) of the housing unit (17, 18, 19, 20, 21).

10. Patient positioning device according to Claim 8 or 9,
**characterized in that** the damping layer (28) is covered by an outer casing (29).

11. Patient positioning device according to one of Claims 8 to 10,
**characterized in that** the damping layer (28) comprises elastic damping material.

12. Patient positioning device according to one of Claims 8 to 11,
**characterized in that** at least one contact sensor (31), in particular a multiplicity of contact sensors (31), is/are disposed in the damping layer (28).

13. Patient positioning device according to one of Claims 1 to 12,
**characterized in that** the robotic arm (2) has at least five, in particular at least six, rotational movement axes (11, 12, 13, 14, 15, 16).

14. Patient positioning device according to Claim 13,
**characterized in that** the robotic arm (2) has a main frame (5), a carousel (6) which is mounted on the main frame (5) so as to be rotatable about a first rotational movement axis (11), a swinging arm (7) which is mounted on the carousel (6) so as to be rotatable about a second rotational movement axis (12), an arm (8) which is rotatably mounted on the swinging arm (7), and a hand (9) which is held on the arm (8), wherein the hand (9) has three rotational movement axes (14, 15, 16).

## Revendications

1. Dispositif de positionnement de patient, en particulier pour la thérapie de capture de neutrons par le bore (BNCT), comprenant un bras de robot (2) et un logement de patient (3) maintenu sur le bras de robot (2),
**caractérisé en ce que** le dispositif de positionnement de patient (1) comprend un agencement de boîtier (4) pour le bras de robot (2), qui comprend au moins une unité (17, 18, 19, 20, 21) formant boîtier qui entoure au moins partiellement le bras de robot (2), l'unité (17, 18, 19, 20, 21) formant boîtier étant fixée sur le bras de robot (2), et **en ce que** le dispositif de positionnement de patient (1) présente un blindage (33) contre les rayonnements ionisants, au moins une partie du blindage (33) étant agencée sur l'unité (17, 18, 19, 20, 21) formant boîtier.

2. Dispositif de positionnement de patient selon la revendication 1,
**caractérisé en ce que** l'unité (17, 18, 19, 20, 21) formant boîtier comprend au moins un couvercle amovible (24) et **en ce qu'**au moins une partie du blindage (33) est maintenue sur le couvercle (24) et est apte à être retirée avec le couvercle (24).

3. Dispositif de positionnement de patient selon la revendication 1 ou la revendication 2,
**caractérisé en ce que** toutes les parties du blindage (33) pour le bras de robot (2) sont fixées à l'agencement de boîtier (4).

4. Dispositif de positionnement de patient selon l'une des revendications 1 à 3,
**caractérisé en ce que** le blindage (33) est agencé sur la face interne (39) de l'unité (17, 18, 19, 20, 21) formant boîtier.

5. Dispositif de positionnement de patient selon l'une des revendications 1 à 4,
**caractérisé en ce que** le bras de robot (2) présente plusieurs sections mobiles les unes par rapport aux autres et **en ce qu'**une unité (17, 18, 19, 20, 21) formant boîtier séparée est prévue pour chaque section.

6. Dispositif de positionnement de patient selon la revendication 5,
**caractérisé en ce qu'**au moins une unité (17, 18, 19, 20, 21) formant boîtier présente un corps de base (23) fixé sur la section et un couvercle (24) maintenu de manière amovible sur le corps de base (23).

7. Dispositif de positionnement de patient selon la revendication 6,
**caractérisé en ce qu'**au moins une unité (17, 18, 19, 20, 21) formant boîtier présente des moyens de positionnement du couvercle (24) par rapport au corps de base (23), notamment un bord périphérique (35).

8. Dispositif de positionnement de patient selon l'une des revendications 1 à 7,
**caractérisé en ce qu'**au moins une unité (17, 18, 19, 20, 21) formant boîtier est pourvue au moins partiellement d'une couche d'amortissement (28).

9. Dispositif de positionnement de patient selon la revendication 8,
**caractérisé en ce que** la couche d'amortissement (28) est maintenue sur une structure porteuse de l'unité (17, 18, 19, 20, 21) formant boîtier, en particulier sur une coque intérieure (27) de l'unité (17, 18, 19, 20, 21) formant boîtier.

10. Dispositif de positionnement de patient selon la revendication 8 ou la revendication 9,
**caractérisé en ce que** la couche d'amortissement (28) est recouverte par une enveloppe extérieure (29).

11. Dispositif de positionnement de patient selon l'une des revendications 8 à 10,
**caractérisé en ce que** la couche d'amortissement (28) comprend un matériau d'amortissement élastique.

12. Dispositif de positionnement de patient selon l'une des revendications 8 à 11,
**caractérisé en ce qu'**au moins un capteur de contact (31), notamment une pluralité de capteurs de contact (31), est agencé dans la couche d'amortissement (28).

13. Dispositif de positionnement de patient selon l'une des revendications 1 à 12,
**caractérisé en ce que** le bras de robot (2) présente au moins cinq, en particulier au moins six axes de mouvement de rotation (11, 12, 13, 14, 15, 16).

14. Dispositif de positionnement de patient selon la revendication 13,
**caractérisé en ce que** le bras de robot (2) comprend un châssis de base (5), un carrousel (6) monté sur le châssis de base (5) de manière à pouvoir tourner autour d'un premier axe de mouvement de rotation (11), un bras oscillant (7) monté sur le carrousel (6) de manière à pouvoir tourner autour d'un deuxième axe de mouvement de rotation (12), un bras (8) monté à rotation sur le bras oscillant (7) et une main (9) maintenue sur le bras (8), la main (9) présentant trois axes de mouvement de rotation (14, 15, 16).
